# EUROPEAN PATENT APPLICATION

(11) **EP 2 348 131 A1**
(43) Date of publication of application: **27.07.2011**
(21) Application number: 10305075.3
(22) Date of filing: 22.01.2010
(51) Int. Cl.: C12Q 1/68

(54) **Low levels of cytidine deaminase as a marker for genetic predisposition to develop cancer**

(71) Applicant: Institut Curie, 75248 Paris Cedex 05 (FR); Centre National de la Recherche Scientifique - CNRS -, 75794 Paris Cedex 16 (FR)
(72) Inventor: Amor-Gueret, Mounira, 94240 L'Hay Les Roses (FR)
(74) Representative: Nargolwalla, Cyra

(57) **Abstract**

The invention relates to methods for detecting a predisposition to develop cancer in a patient comprising the step of detecting the level of cytidine deaminase (CDA) in a biological sample obtained from said patient. The invention also relates to the use of uridine analogs for preventing cancer.

SEQUENCE LISTING

<110>  Institut Curie

<120>  Low levels of cytidine deaminase as a marker for genetic
       predisposition to develop cancer

<130>  0930-PR

<160>  12

<170>  PatentIn version 3.3

<210>  1
<211>  19
<212>  DNA
<213>  artificial

<220>
<223>  primer

<400>  1
ctgatgccga ctggaggtg                                                    19


<210>  2
<211>  20
<212>  DNA
<213>  artificial

<220>
<223>  primer

<400>  2
tgacaacagt gaccccagga                                                   20


<210>  3
<211>  20
<212>  DNA
<213>  artificial

<220>
<223>  primer

<400>  3
cccctacagt cactttcctg                                                   20


<210>  4
<211>  20
<212>  DNA
<213>  artificial

<220>
<223>  primer

<400>  4
cgggtagcag gcattttcta                                                   20


<210>  5
<211>  20
<212>  DNA
<213>  artificial

<220>
<223>  primer

<400>  5
gaccagtcaa caggggacat                                                   20


<210>  6
<211>  24
<212>  DNA
<213>  artificial

<220>
<223>  primer

<400>  6
aacacttcgt ggggtccttt tcac                                              24


<210>  7
<211>  22
<212>  DNA
<213>  artificial

<220>
<223>  primer

<400>  7
gcattgacaa cagggttcgt ag                                                22


<210>  8
<211>  22
<212>  DNA
<213>  artificial

<220>
<223>  primer

<400>  8
gcggttcttg gaggaaacat tg                                                22


<210>  9
<211>  16
<212>  DNA
<213>  artificial

<220>
<223>  primer

<400>  9
ggcaatgcgg ctgcaa                                                       16


<210>  10
<211>  19
<212>  DNA
<213>  artificial

<220>
<223>  primer

<400>  10
gggtacccac gcgaatcac                                                    19


<210>  11
<211>  20
<212>  DNA
                                  Page 2

<213>  artificial

<220>
<223>  primer

<400>  11
tgggaacaag agggcatctg                                                    20


<210>  12
<211>  22
<212>  DNA
<213>  artificial

<220>
<223>  primer

<400>  12
ccaccactgc atcaaattca tg                                                 22

## Description

### FIELD OF THE INVENTION

The invention relates to methods for detecting a genetic predisposition to develop cancer and methods for preventing cancer.

### BACKGROUND OF THE INVENTION

Cancer is a class of diseases in which a group of cells display the traits of uncontrolled growth (growth and division beyond the normal limits), invasion (intrusion on and destruction of adjacent tissues), and sometimes metastasis (spread to other locations in the body via lymph or blood). Cancers can be classified according to the organ, tissue and cell-type from which the cancerous cells originate: lung, colon, liver, skin etc.

Cancer represents one of the leading causes of death in the world. Successful treatment relies on the diagnosis of the disease at very early stages. A plurality of risk factors (lifestyle related, genetic etc.) has been identified for certain types of cancers. For example, mutations in the *BRCA1* and *BRCA2* genes have been associated with increased risk of developing breast cancer. Similarly, several genes have been identified which are linked to a predisposition to develop colorectal cancer.
However, to date, there is no universal marker of the genetic instability associated with the pre-tumoral state, which may serve as a marker for genetic predisposition to develop any type of cancer.
Thus, there is still an unfulfilled need in the art for methods for predicting the risk of developing a cancer in a patient.

Cytidine deaminase (CDA) is an enzyme involved in pyrimidine salvaging. It irreversibly deaminates cytidine and deoxycytidine to their uridine counterparts. It is one of several deaminases responsible for maintaining the cellular pyrimidine pool.
Mutations in the gene encoding CDA are associated with toxicity of certain agents known as anti-metabolites used in the treatment of certain cancers. Indeed, in addition to native nucleotides, CDA also deaminates and therefore inactivates cytotoxic nucleoside analogues 5'-aza-cytidine, 2',2'-difluorodeoxycytidine (gemcitabine) and cytosine arabinoside (Ara-C) that are effective chemotherapeutic agents widely used in the treatment of different types of cancer. However, some cancer patients treated with such drugs over-react to the chemotherapy due to a hypersensitivity to the drugs resulting from a low level of CDA activity (and thus from a reduced drug inactivation).
Conversely, CDA activates certain chemotherapeutic drugs such as capecitabine (which is a pro-drug which is then converted inter alia by CDA into the active drug 5-fluorouracil). Thus, CDA levels are known from the prior art to correlate with sensitivity and/or toxicity of certain chemotherapeutic anti-metabolite agents (see for example WO2009/021551). However, no prior art document has ever established a link between CDA levels and predisposition to cancer.

There is still a need in the art for reliable, measurable biomarkers having a good predictive value for the risk of developing cancer in a patient.

### SUMMARY OF THE INVENTION

The inventors have discovered that low levels of cytidine deaminase (CDA) are associated with genetic instability known to be associated with an increased risk of developing a cancer.

Hence, in one aspect, the invention relates to a method for detecting a predisposition to cancer in a patient comprising the step of detecting the level of CDA in a biological sample obtained from said patient.

The invention also relates to a kit for detecting a predisposition to cancer in a patient, comprising means for detecting the level of CDA in a biological sample obtained from said patient.

The invention also relates to the use of a kit for detecting the level of CDA in a biological sample from a patient for detecting a predisposition to cancer in said patient.

In another aspect, the invention also relates to an agent which increases the level of deoxyuridine for use in a method for preventing cancer in a patient.

### DETAILLED DESCRIPTION OF THE INVENTION

### Diagnostic methods and kits of the invention

The inventors have discovered that low levels of CDA are associated with genetic instability known to be associated with an increased risk of developing a cancer. Indeed, the inventors showed that low levels of CDA result in an increase in the frequency of sister chromatid exchanges (SCEs). An elevation of the levels of SCEs is pathognomonic of the Bloom syndrome, known to predispose patients to all types of cancers affecting the general population. Moreover, increases in SCEs levels in mammals have been used extensively to identify DNA-damaging carcinogens.

Hence, in one aspect, the invention relates to a method for detecting a predisposition to cancer in a patient comprising the step of detecting the level of cytidine deaminase in a biological sample obtained from said patient.
Typically, the invention relates to a method for detecting a predisposition to cancer in a patient comprising the step of detecting the level of cytidine deaminase in a biological sample obtained from said patient, wherein a decreased level of cytidine deaminase compared to the normal level is indicative of a predisposition to cancer.

The invention also relates to a kit for detecting a predisposition to cancer in a patient, comprising means for detecting the level of CDA in a biological sample obtained from said patient.
In a further aspect, the invention relates to a kit for detecting a predisposition to cancer in a patient comprising means for measuring the level of CDA in a biological sample obtained from said patient and a leaflet describing instructions for use, wherein the leaflet indicates that a low level of CDA is indicative of a risk of developing cancer.

The invention also relates to the use of a kit for detecting the level of CDA in a biological sample from a patient for detecting a predisposition to cancer in said patient.

As used herein, the term "patient" denotes a mammal, such as a rodent, a feline, a canine, a bovine, an equine, a sheep, a porcine and a primate. Preferably, a patient according to the invention is a human.

The biological sample suitable for carrying out the invention may be a body fluid, such as serum, plasma, blood or urine. It may also be a tissue biopsy of any kind, such as a breast biopsy or a liver biopsy.
In a preferred embodiment, the biological sample is serum. Said serum can be freshly collected serum or frozen serum.

As used herein, the expression "Cytidine deaminase" or "CDA", also known as "CDD" refers to the enzyme which catalyzes the deamination of cytidine and deoxycytidine to their uridine counterparts. CDA is known as EC 3.5.4.5 under the IUBMB Enzyme Nomenclature. "CDA" can encompass CDA of any mammalian origin, such as preferably primate, even more preferably human.
Typically, CDA according to the invention is the enzyme encoded by the gene located on chromosome 1, at locus lp36.2-p35 of the human genome.

As used herein, the expression 'level of Cytidine deaminase" or "level of CDA" has its general meaning in the art. It can refer to the enzymatic activity of CDA, to the amount of CDA protein or the amount of mRNa encoding CDA in said biological sample As used herein, a "decreased level of CDA" is a level of CDA which is lower than that observed in the general population. A level of CDA is deemed to be lower than the general population, when it is lower than the normal by a factor 1.5, preferably 2, even more preferably 2, 3...10, or when it is not detectable.

In one embodiment, the level of CDA can refer to level of CDA enzymatic activity, i.e. to the capacity to deaminate Cytidine or deoxycytidine to uridine or deoxyuridine respectively.
In one aspect, the invention therefore relates to a method for detecting a predisposition to cancer in a patient comprising the step of detecting the level of CDA enzymatic activity in a biological sample obtained from said patient.

Typically, the enzymatic activity of CDA can be measured according to available enzymatic tests. Suitable enzymatic tests of CDA levels can include, but are not limited to:
- assays measuring the conversion of radiolabelled cytidine to uridine (see for example the test described in document WO02092062 on p11),
- assays measuring the amount of 5'-dFUrd produced from 5'-dFCrd by HPLC (see for example Watanabe et al., Expression of cytidine deaminase in human solid tumors and its regulation by 1α,25-dihydroxyvitamin D3, Biochimica et Biophysica Acta, 1312, 1996, 99-104),
- spectrophotometric methods, based upon the release and detection of ammonium from cytidine (see WO09021551 and references therein, and Ciccolini et al., J. Clin. Oncol., 28, 2010, 160).

Typically, the level of CDA activity is deemed to be decreased if the level of CDA activity measured the patient's serum is below a certain threshold. Typically, the level of CDA activity is deemed to be decreased if the level of CDA activity measured the patient's serum is below about 1.4 U/mg, preferably below about 1.3 U/mg, even more preferably below about 1.2 U/mg, below about 1.1 U/mg, below about 1.0 U/mg, below about 0.9 U/mg, below about 0.8 U/mg; wherein 1U corresponds to 4.10⁻³ µMol of ammonium released per minute and per milliliter of serum according to the method developped by Ciccolini et al (J. Clin. Oncol., 28, 2010, 160).

In one aspect, the invention relates to a kit for detecting a predisposition to cancer in a patient comprising means for measuring the level of CDA activity in a biological sample obtained from said patient.
Typically, said kit can comprise:
i. a container comprising cytidine;
ii. a contained comprising ammonium;
iii. optionally, a leaflet which describes a method for determining the CDA activity in a biological sample, by measuring the amount of ammonium released through conversion of cytidine to uridine by spectrophotometry, wherein the leaflet indicates that a low CDA activity (for example inferior to about 1.2 U/mg), in a serum sample, is indicative of a risk of developing a cancer.

The invention also relates to the use of such a kit for detecting the level of CDA in a biological sample obtained from a patient for detecting a predisposition to cancer in said patient.

In another embodiment, the level of CDA is the level of the CDA protein found in the biological sample.

In one aspect, the invention therefore relates to a method for detecting a predisposition to cancer in a patient comprising the step of detecting the level of the CDA protein in a biological sample obtained from said patient.

In a particular embodiment, the methods of the invention comprise contacting the biological CDA protein present in the biological sample. The binding partner may be an antibody that may be polyclonal or monoclonal, preferably monoclonal. In another embodiment, the binding partner may be an aptamer.
Polyclonal antibodies of the invention or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred.
Monoclonal antibodies of the invention or a fragment thereof can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985). Alternatively, techniques described for the production of single chain antibodies (see e.g. U.S. Pat. No. 4,946,778) can be adapted to produce anti-CDA single chain antibodies. Antibodies useful in practicing the present invention also include anti-CDA fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to CDA. For example, phage display of antibodies may be used. In such a method, single-chain Fv (scFv) or Fab fragments are expressed on the surface of a suitable bacteriophage, e. g., M13. Briefly, spleen cells of a suitable host, e. g., mouse, that has been immunized with a protein are removed. The coding regions of the VL and VH chains are obtained from those cells that are producing the desired antibody against the protein. These coding regions are then fused to a terminus of a phage sequence. Once the phage is inserted into a suitable carrier, e. g., bacteria, the phage displays the antibody fragment. Phage display of antibodies may also be provided by combinatorial methods known to those skilled in the art. Antibody fragments displayed by a phage may then be used as part of an immunoassay.

Antibodies against CDA are available for example from Abcam under the references ab56053, ab78231, ab82347 and ab82346.

In another embodiment, the binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in Tuerk C. 1997. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A, that are selected from combinatorial libraries by two hybrid methods (Colas et al., 1996).

The binding partners of the invention, such as antibodies or aptamers, may be labelled with a detectable molecule or substance, such as a fluorescent molecule, a radioactive molecule or any others labels known in the art. Labels are known in the art that generally provide (either directly or indirectly) a signal.
As used herein, the term "labelled", with regard to the antibody, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody or aptamer, as well as indirect labelling of the probe or antibody by reactivity with a detectable substance. An antibody or aptamer of the invention may be labelled with a radioactive molecule by any method known in the art. For example radioactive molecules include but are not limited radioactive atom for scintigraphic studies such as I123, I124, In111, Re186, Re188.

The aforementioned assays generally involve the binding of the binding partner (ie. antibody or aptamer) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

The level of the CDA protein may be measured by using standard immunodiagnostic techniques, including immunoassays such as competition, direct reaction, or sandwich type assays. Such assays include, but are not limited to, agglutination tests; enzyme-labelled and mediated immunoassays, such as ELISAs; biotin/avidin type assays; radioimmunoassays; immunoelectrophoresis; immunoprecipitation.
More particularly, an ELISA method can be used, wherein the wells of a microtiter plate are coated with a set of antibodies against CDA. The biological sample is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art.

In one aspect, the invention relates to a kit for detecting a predisposition to cancer in a patient comprising means for measuring the level of CDA protein in a biological sample obtained from said patient.
Typically, said kit can comprise antibodies against CDA or aptamers against CDA.

In an alternative embodiment, the level of CDA can be measured by measuring the amount of messenger RNA (mRNA) encoding CDA.
In one aspect, the invention therefore relates to a method for detecting a predisposition to cancer in a patient comprising the step of detecting the level of mRNA encoding CDA in a biological sample obtained from said patient.

Typically, said method can comprise a step of isolating total RNA or total mRNA from said biological sample, prior to the detection of the level of mRNA encoding CDA.
The skilled person in the art knows how to carry out such isolating steps using standard procedures.

Methods for detecting the presence of mRNA are well known in the art. For example the nucleic acid contained in the samples is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). In a preferred embodiment, the expression of the *CDA* gene or is detected by RT-PCR, preferably quantitative or semi-quantitative RT-PCR, even more preferably real-time quantitative or semi-quantitative RT-PCR.
Other methods of amplification include ligase chain reaction (LCR), transcription-mediated amplification (TMA), strand displacement amplification (SDA) and nucleic acid sequence based amplification (NASBA).
Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).
Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).
Suitable oligonucleotides for measuring CDA mRNA are for example:
Forward sequence: CCCCTACAGTCACTTTCCTG (SEQ ID No:3)
Reverses sequence: CGGGTAGCAGGCATTTTCTA (SEQ ID No:4).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.
In a preferred embodiment, said kit comprises oligonucleotides for determining the level of CDA mRNA by quantitative PCR. Typically, said kit can comprise oligonucleotides specific for the CDA mRNA and internal normalization oligonucleotides (housekeeping genes). Suitable oligonucleotides for measuring CDA mRNA are for example:
Forward sequence: CCCCTACAGTCACTTTCCTG (SEQ ID No:3)
Reverses sequence: CGGGTAGCAGGCATTTTCTA (SEQ ID No:4).

In a further embodiment, the level of CDA can be assayed indirectly by genotyping the *CDA* gene. Indeed, it has been shown that certain mutations or polymorphisms in the gene encoding CDA are associated with altered levels of CDA activity. For instance, the 79A>C single mutation, the canonical 208A>G polymorphism have been described. Typically, these polymorphisms have been identified for example in relation with drug-exposure toxicities such as gemcitabine toxicity.
In one aspect, the invention therefore relates to a method for detecting a predisposition to cancer in a patient comprising the step of detecting the level of CDA in a biological sample obtained from said patient, wherein said level of CDA is assayed indirectly by genotyping the *CDA* gene.
Typically, said method can comprise a step of isolating DNA from said biological sample, prior to the detection of mutations in the gene encoding CDA.
The skilled person in the art knows how to carry out such isolating steps using standard procedures.

A decreased expression of the *CDA* gene resulting in lower levels of CDA-encoding mRNA and/or lower levels of CDA protein can be due to mutations in the CDA promoter or in the CDA coding sequence. Alternatively, certain decreased activity of CDA can be due to mutations in the coding sequence which do not influence the expression levels.

As used herein, the expression "predisposition to cancer" refers to a patient's susceptibility or proneness to develop a cancer of any type. Said predisposition to cancer may be purely hereditary (inherited mutation for example) or acquired (spontaneous mutations, epigenetic regulations etc.). Predisposition to cancer can be defined as an increased risk of developing a cancer, when compared to the general population.

As used herein, the term "cancer" has its general meaning in the art. It refers to the pathological condition in mammals that is characterized by unregulated cell growth. Examples of cancer include, but are not limited to solid tumors or a carcinoma. Preferably, the solid tumor is selected from breast cancer, colon cancer, lung cancer, prostate cancer, renal cancer, metastatic or invasive malignant melanoma, brain tumor, bladder cancer and liver cancer. Carcinoma includes bladder, breast, colon, kidney, liver, lung, ovary, pancreas, stomach, cervix, thyroid or skin carcinoma, including squamous cell carcinoma. However, the present invention also contemplates hematopoietic tumors such as leukemia, acute lymphocytic leukemia, acute lymphoblastic leukemia, B-cell lymphoma, T-cell lymphoma, Hodgkins lymphoma, non- Hodgkins lymphoma, hairy cell lymphoma, Burketts lymphoma, acute and chronic myelogenous leukemias and promyelocytic leukemia.

In a preferred embodiment, said cancer is selected from the group consisting of breast cancer, lung cancer, colon cancer, prostate cancer and acute myeloid leukemia.

### Prophylactic methods and pharmaceutical compositions of the invention

In another aspect, the invention provides methods for preventing cancer, comprising the administration of a therapeutically effective amount of an agent which increases the level of deoxyuridine (the reaction product of CDA) in order to compensate for low levels of CDA.

Thus, the invention also relates to an agent which increases the level of deoxyuridine for use in a method for preventing cancer in a patient.

Typically, the agent which increases the level of deoxyuridine is used for preventing cancer in a patient who has a low level of CDA, such as a patient who has been identified as at risk by the method for determining predisposition to cancer described above.

As used herein, the terms "prevention", "prevent", "preventing" refer to the fact of stopping/delaying the occurrence of cancer, reducing of the risk of cancer, or slowing down the development of said cancer. It can also refer to the prevention or slowing down of one or more symptoms of cancer (such as the occurrence of metastasis).

By a "therapeutically effective amount" of an agent which increases the level of deoxyuridine is meant a sufficient amount to prevent cancer, at a reasonable benefit/risk ratio applicable to any medical treatment. It will be understood, however, that the total daily usage of an agent which increases the level of deoxyuridine will be decided by the attending physician within the scope of sound medical judgment. The specific therapeutically effective dose for any particular subject in need thereof will depend upon a variety of factors including other cancer predisposition markers, lifestyle-related risk factors and the activity of the specific agent which increases the level of deoxyuridine to be used, the age, body weight, general health, sex and diet of the subject, the time of administration, route of administration, the duration of the treatment; drugs used in combination or coincidental with the and like factors well known in the medical arts.

In one embodiment, said agent which increases the level of deoxyuridine is an agent capable of restoring the level of the CDA protein (recombinant protein, expression vectors etc.).
In one aspect, the invention therefore relates to an agent capable of restoring the level of the CDA protein for use in a method for preventing cancer in a patient.
Typically, said agent capable of restoring the level of the CDA protein may be the CDA protein itself. Said CDA protein may be a purified or a recombinant protein. Methods for producing recombinant proteins are well known in the art.
Alternatively, said agent of restoring the level of the CDA protein may be an expression vector comprising the gene encoding CDA. Successful gene transfer studies have shown that is possible to restore levels of CDA in this way (see for example Bardenhauer et al., Resistance to cytarabine and gemcitabine and in vitro selection of transduced cells after retroviral expression of cytidine deaminase in human hematopoietic progenitor cells. Leukemia, 2005, 19(12), 2281-8.; Rattmann et al. Gene transfer of cytidine deaminase protects myelopoiesis from cytidine analogs in an in vivo murine transplant model, Blood, 2006, 108, 2965-2971, and for review Hébrard et al., Development of gene therapy in association with clinically used cytotoxic deoxynucleoside analogues, Cancer Gene Ther. 2009, 16(7), 541-50.)
As used herein, the terms "expression vector" refer to a nucleic acid molecule capable of directing the expression of a given nucleic acid sequence which is operatively linked to an expression control sequence or promoter. In particular, an expression vector according to the invention is a vector which enables the expression of a given nucleic acid sequence into the protein encoded by said nucleic acid sequence in a eukaryotic host cell. The promoter of said expression vector is typically a eukaryotic promoter.
The expression vector(s) of the present invention can be a plasmid or a viral vector. A plasmid is a circular double-stranded DNA loop that is capable of autonomous replication. A viral vector is a nucleic acid molecule which comprises viral sequences which can be packaged into viral particles. A variety of viral vectors are known in the art and may be adapted to the practice of this invention, including e.g., adenovirus, AAV, retrovirus, hybrid adeno-AAV, lentivirus and others. By carrying out routine experimentation, the skilled person in the art can chose from the variety of available vectors, those which are suitable for carrying out the method of the invention.
In one embodiment said agent capable of restoring the level of the CDA protein is not vitamin D.

In another embodiment, said agent which increases the level of deoxyuridine is a uridine analog.
In one aspect, the invention therefore relates to a uridine analog for use in a method for preventing cancer in a patient.
As used herein, the expression "uridine analog" refers to a compound which bares structural similarity with uridine, or a metabolic precursor thereof. The uridine analog of the invention is not a cytostatic nor a cytotoxic analog. In other words, it is not an antimetabolite (compound which resembles uridine in that it can be incorporated into ribonucleic or desoxyribonucleic acid but which induces cell cycle arrest as a consequence of this incorporation).
Suitable uridine analogs according to the invention include, but are not limited to:
- uracil, uridine, uridine monophosphate, uridine biphosphate, uridine triphosphate;
- thymine, thymidine, thymidine monophosphate, thymidine biphosphate, thymidine triphosphate;
- β-D-uridine-5'-bis(SATE)phosphotriester, a prodrug of β-D-uridine-5'-monophosphate (Faraj et al., Selective protection of toxicity of 2',3'-dideoxypyrimidine nucleoside analogs by β-D-uridine in human granulocyte-macrophage progenitor cells. Antiviral Research, 1996, 29, 261-267);
- acylated uridine nucleosides such as those described in EP 1491201 B1, and in particular triacetyluridine (TAU), also known as vistonuridine or PN401 (from Wellstat Therapeutics).
- 5-(phenylthio)acyclouridine (PTAU) as described in Omar et al., 5-(phenylthio)acyclouridine: a powerful enhancer of uridine bioavailability: relevance to chemotherapy with 5-fluorouracil and other uridine rescue regimes, Cancer Chemother Pharmacol, 2005, 55, 541-551.
- any other uridine analog which can be used for uridine rescue regimens.

In a preferred embodiment, the uridine analog according to the invention is selected from the group consisting of uracil, uridine, uridine monophosphate, uridine biphosphate, uridine triphosphate, thymine, thymidine, thymidine monophosphate, thymidine biphosphate, thymidine triphosphate, β-D-uridine-5'-bis(SATE)phosphotriester, triacetyluridine and 5-(phenylthio)acyclouridine.

The uridine analog may be combined with pharmaceutically acceptable excipients, and optionally sustained-release matrices, such as biodegradable polymers, to form therapeutic compositions.
"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.
In the pharmaceutical compositions of the present invention for oral, sublingual, subcutaneous, intramuscular, intravenous, transdermal, local or mucosal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.
Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.
The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.
Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.
The uridine analog of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.
The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with one or several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.
Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.
For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.
In addition to the compounds of the invention formulated for parenteral administration, such as intravenous or intramuscular injection, other pharmaceutically acceptable forms include, e.g. tablets or other solids for oral administration; liposomal formulations; time release capsules; and any other form currently used.

In a preferred embodiment, the uridine analog is administered in combination with another active agent.
In one embodiment, the uridine analog of the invention is used in combination with vitamin D. The use of vitamin D to prevent cancer has been described previously (for review see for example Hines et al., Breast cancer survivors and vitamin D: a review, Nutrition, 2010, p1-8). In addition, vitamin D has been shown to upregulate CDA expression in certain cell lines (see for example document WO02/092062 and Watanabe et al. Expression of cytidine deaminase in human solid tumors and its regulation by 1α,25-dihydroxyvitamin D₃.)

Typically, the uridine analog can be administered in combination with an agent used to prevent cancer, such as anti-oxidant agent. Suitable anti-oxidant agents include, but are not limited to, natural antioxidants such as ascorbic acid (AA, E300) and tocopherols (E306), as well as synthetic antioxidants such as propyl gallate (PG, E310), tertiary butylhydroquinone (TBHQ), butylated hydroxyanisole (BHA, E320) and butylated hydroxytoluene (BHT, E321). Typically, the uridine analog and the other active agent can be formulated separately. Alternatively, they can be formulated together in a pharmaceutical composition.

The invention will be further described by the following examples and figures, which are not intended to limit the scope of the protection defined by the claims.

### Figure legends

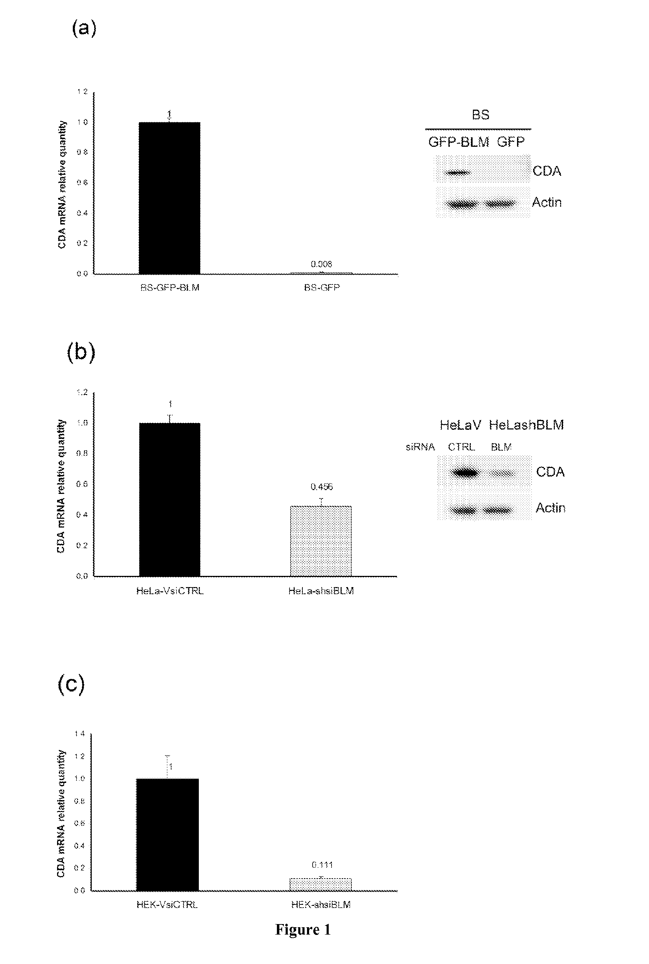

**Fig. 1**. CDA expression is downregulated in BLM-deficient cells. CDA mRNA expression was determined by quantitative Real-Time PCR on: (a) BS-GFP cell line compared to its control BS-GFP-BLM cell line, (b) HeLash-siBLM cell line compared to its control HeLaV-siCTRL and (c) HEK293sh-siBLM cell line compared to its control HEK293V-siCTRL cell line. For each cell line, CDA mRNA expression was monitored in 3 independant samples and normalized with 4 reference genes. Errors bars represent standard error of the mean. CDA protein level was assessed by immunoblotting on (a) BS-GFP and BS-GFP-BLM cells (b) HeLash-siBLM and HeLaV-siCTRL cells.

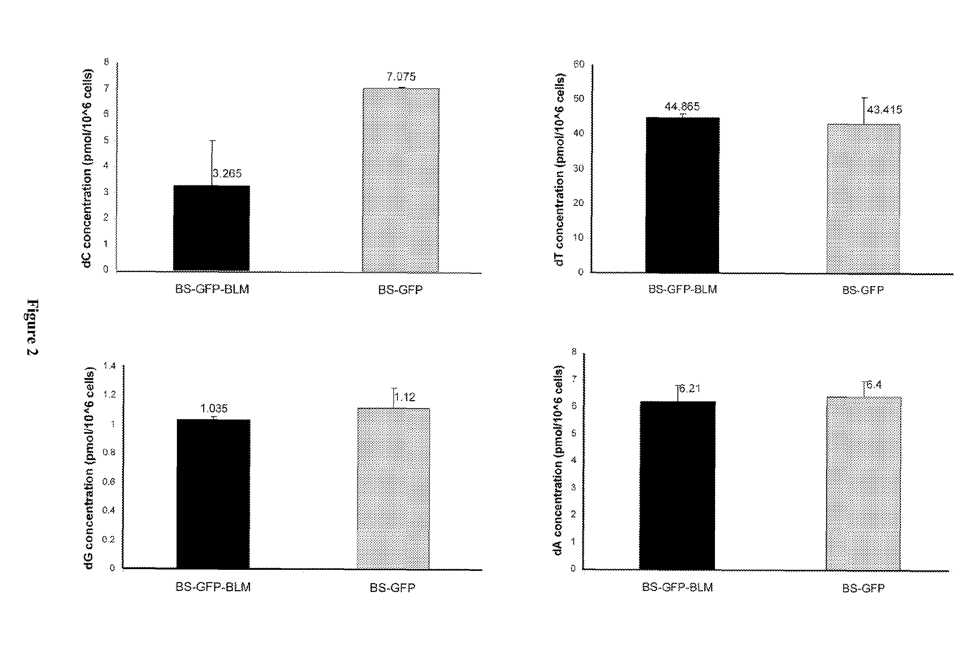

**Fig. 2**. dCTP pool is significantly increased in BLM-deficient cells. The 4 deoxyribonucleoside triphosphates (dNTP) pool were measured with 20 to 30 x10⁶ cells using an enzymatic assay in BS-GFP-BLM and BS-GFP cells. Results are given in pmol for 10⁶ cells. The means of 2 independent experiments are shown, and errors bars represent standard error of the mean.

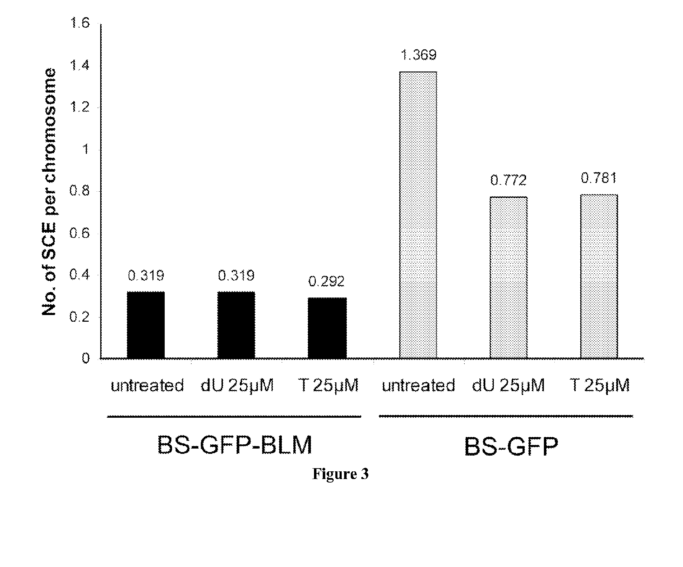

**Fig. 3****.** Culturing BS cells in the presence of dU or T decreases SCE level. Number of SCE per chromosome were determined for BS-GFP-BLM and BS-GFP cell lines left untreated or treated with 25 µM dU or 25 µM T for 48 h before adding BrdU, followed by 48 h in the presence of BrdU. About 600 chromosomes were analyzed for each condition.

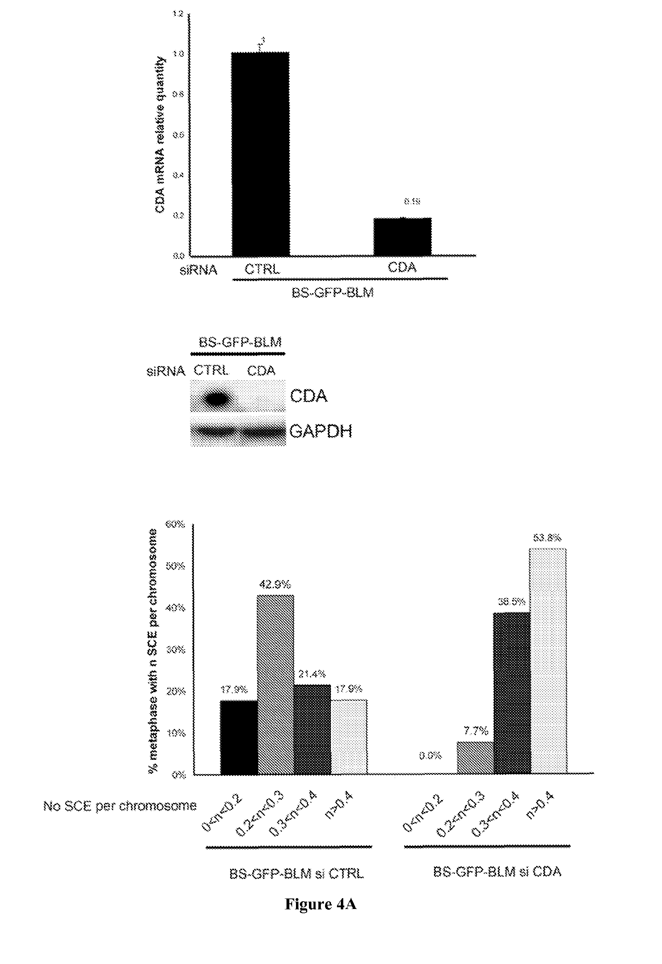

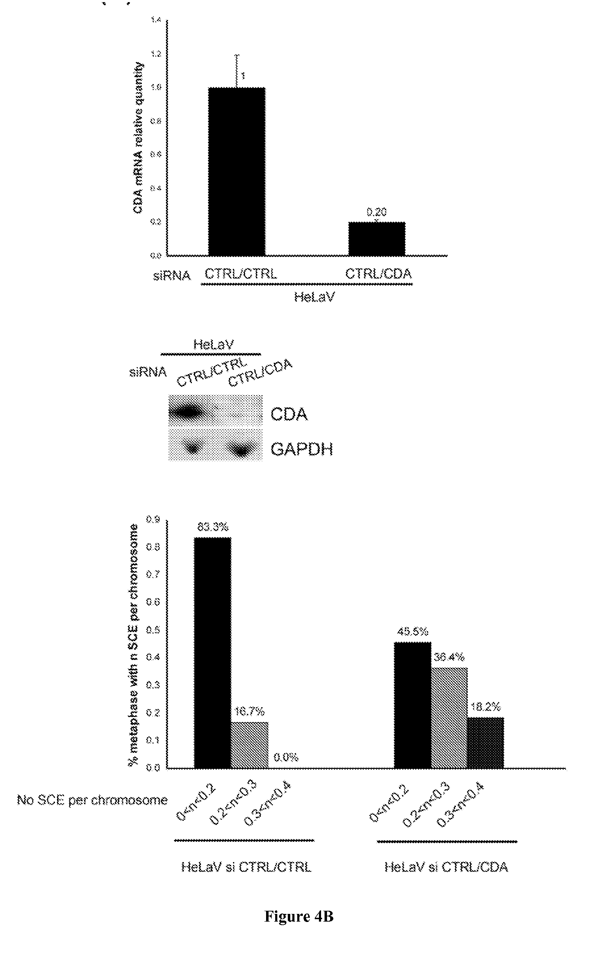

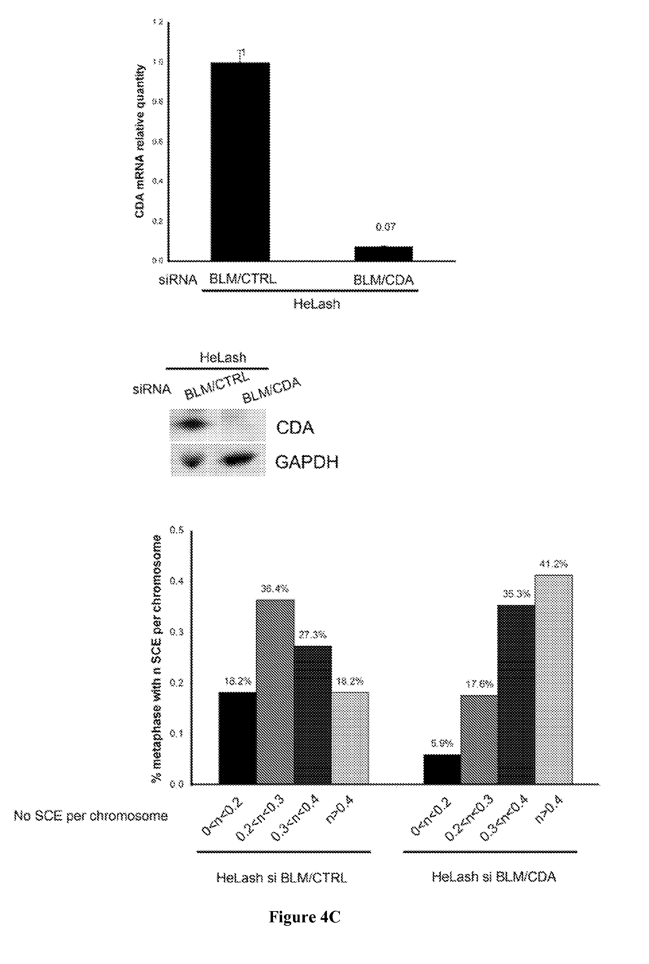

**Fig. 4****.** siRNA-mediated depletion of CDA is associated with a significant increase in SCE frequency. BS-GFP-BLM cells were transfected with a pool of siRNA CTRL or siRNA CDA. HeLaV cells were co-transfected with a pool of siRNA CTRL/CTRL or siRNA CTRL/CDA and HeLashBLM cells were co-transfected with siRNA BLM/CTRL or siRNA BLM/CDA. In transfected cells, CDA mRNA expression was measured by quantitative Real-Time PCR and CDA protein level was assessed by immunoblotting : (a) BS-GFP-BLM cells - upper and middle panel, (b) HeLaV cells - upper and middle panel, (c) HeLash - upper and middle panel. SCE assays were performed on transfected cells and metaphases captured were sorted by number of SCE per chromosome. For each condition, 20 to 30 metaphases from 2 independent experiments were sorted. Metaphases proportions for 4 ranges of SCE level were calculated: (a) BS-GFP-BLM cells - lower panel, (b) HeLaV cells - lower panel, (c) HeLash cells - lower panel.

### EXAMPLES

### Example 1: Low CDA levels are associated with genetic instability

The following example demonstrates, in a cellular model of Bloom syndrome (BS), that low CDA levels are associated with genetic instability and with a predisposition to developing cancer. Culturing BS cells in the presence of deoxyuridine reduce significantly their genetic instability. This further demonstrates that patients with low levels of CDA can be treated with uridine analogs.

Bloom syndrome (BS) is a rare human autosomal recessive disorder characterized by marked genetic instability associated with a greatly increased predisposition to a wide range of cancers commonly affecting the general population.
Cells from BS patients have a mutator phenotype and display many cytogenetic abnormalities including increases of chromosome breaks; symmetric quadriradial chromatid interchanges between homologous chromosomes and sister chromatid exchanges (SCEs). The hallmark of BS cells and the only criterion for BS diagnosis is the approximately 10 times higher frequency of SCEs in BS cells than in normal cells (for a review on BS, see Amor-Guéret, 2006, Cancer Lett., 236, 1-12). BS cells can be considered to be a good cellular model for pre-tumoral states.

### Materials and methods

### Cell cultures and transfections

HeLa cells were used, as previously described (Dutertre S et al., Oncogene 19, 2731-8, 2000). HEK293 cells were cultured as HeLa cells.
HeLaV cells and HeLashBLM cells, HEK293V and HEK293shBLM were obtained by transfecting HeLa and HEK cells with an empty pSM2 vector or a pSM2 vector encoding a short hairpin RNA sequence directed against BLM (Open Biosystems, clone V2HS-89234), respectively, using JetPEI reagent (Ozyme) according to the manufacturer's instructions. After 48 hours, selection with 1 to 5 µg/ ml puromycin (Invivogen) was applied for HeLa cells and with 7 to12 µg/ml puromycin for HEK cells. Individual colonies were isolated and maintained in growth medium containing 0.5 µg/ ml puromycin for Hela cells or 10µg/ml puromycin for HEK cells.
For siRNAs transient transfection assays, 3-4 x 10⁵ cells were seeded in 3 ml of medium in each well of a six-well plate. Cells were transfected with siRNAs specific for BLM or CDA (ON-TARGETplus, SMARTpool, Dharmacon), or negative control siRNAs (ON-TARGETplus siCONTROL Non Targeting Pool, Dharmacon) at a final concentration of 100 nM (single transfections) or 200 nM (co-transfections) for 48 to 72 hours, using DharmaFect I™ (Dharmacon), according to the manufacturer's instructions. For siCDA transfections, the experiment was performed twice successively for a global time of 120 hours.

The BS SV40-transformed fibroblast cell line GM08505B was cultured as previously described (Dutertre S et al., Oncogene 19, 2731-8, 2000). BS-GFP-BLM cells were obtained by transfecting BS GM08505B cells with an empty EGFP-C1 vector (Clontech, Mountain View, CA), or a EGFP-C1 vector containing the full length BLM cDNA (Eladad et al., Hum Mol Genet 14, 1351-65, 2005), respectively, using JetPEI reagent (Ozyme). After 48 hr, selection with 800 to 1600 µg/ml of G418 (Invitrogen) was applied. Individual colonies were isolated and maintained in DMEM supplemented with 800 µg/ml G418. SCE analysis showed that BS cells stably expressing GFP-BLM were efficiently complemented (data not shown).

All cell lines were cultured in DMEM with 10% fetal calf serum (PAN laboratories) either not dialyzed (Lot P282710) or dialyzed (Lot P5940309) for nucleoside complementation experiments and dNTP assays.

### Culture in presence of deoxynucleosides

Deoxynucleosides were obtained from Sigma, and added to medium at a concentration of 25µM for 2 x 48 hours.

### RNA extraction and cDNA synthesis

Total RNA were purified from 2 to 3 x 10⁶ cells using the RNeasy Mini kit (Qiagen) according to the manufacturer's instructions. A DNAse digestion step was performed as indicated in the protocol.
RNA quality was tested using Experion system (Biorad) and 28S/18S ratio was calculated. If the ratio >1.8, RNA were used for cDNA synthesis. The cDNA were synthesized using 250 ng of random hexamers (Invitrogen), 2 µg RNA and Superscript II reverse transcriptase (Invitrogen) according to manufacturer's instructions.

### Quantitative Real-Time PCR experiments

The experiments were performed according to the MIQE Guideline (Bustin et al., Clin Chem 55, 611-22, 2009). For each cell line, 3 cDNAs issued from 3 independent RNA extracts were used for quantification. Amplification mixtures (10µ1) contained template cDNA (1/100 final dilution), iQ SYBR Green Supermix 1x (BioRad) and 300 nM forward and reverse primer (See sequences below). Amplification was performed with CFX96 detection system (BioRad) in adapted 96 wells-plate (BioRad) and cycling conditions comprised 3 minutes polymerase activation at 95°C, and 40 cycles at 95° for 10 seconds and 60°C for 30 seconds. For each sample and primer set, the reactions were performed in triplicate. All PCR efficiencies were above 95% and below 102%.
The relative quantities of CDA and BLM mRNAs were normalized, using CFX manager software (BioRad), against 4 reference genes mRNAs, chosen because of their low M-value (Vandesompele et al., Genome Biol 3, 2002 - Calculated by the software).

### Primer sequences

| **Symbol** | **Forward primer** | **Reverse primer** |
|---|---|---|
| **BLM(1)** | CTGATGCCGACTGGAGGTG (SEQ ID No: 1) | TGACAACAGTGACCCCAGGA (SEQ ID No:2) |
| **CDA** | CCCCTACAGTCACTTTCCTG (SEQ ID No:3) | CGGGTAGCAGGCATTTTCTA (SEQ ID No:4) |
| **HPRT1* (2)** | GACCAGTCAACAGGGGACAT (SEQIDNo:51) | AACACTTCGTGGGGTCCTTTTCAC (SEQ ID No:6) |
| **RPL32* (2)** | GCATTGACAACAGGGTTCGTAG (SEQ ID No:7) | GCGGTTCTTGGAGGAAACATTG (SEQ ID No:8) |
| **HMBS* (3)** | GGCAATGCGGCTGCAA (SEQ ID No:9) | GGGTACCCACGCGAATCAC (SEQ ID No:10) |
| **SDHA* (3)** | TGGGAACAAGAGGGCATCTG (SEQ ID No:11) | CCACCACTGCATCAAATTCATG (SEQ ID No:12) |

| | | |
|---|---|---|
| * Internal control genes (1) Sequence described in Bischof et al. 153, 367-80, 2001 (2) Sequence described in Viegas et al., Nucleic Acids Research 35, 4542-51, 2007 (3) Sequence described in Vandesompele et al., Genome Biol 3, 2002 | | |

### Western Blot analysis

Cells were lysed in 350 mM NaCl, 50 mM Tris-HCl pH7.5, 1% NP-40 and proteases inhibitors (Roche), sonicated and heated at 70°C for 10 minutes. Samples equivalent to 15 µg of protein were subjected to NuPAGE Novex 4-12 % Bis-Tris pre-cast gels (Invitrogen). The procedure for immunoblotting was described in Ababou et al., Oncogene 21, 2079-88, 2002. All the commercial antibodies were used according to the manufacturer's instructions. The primary antibodies used against BLM were ab476 (1:1000; rabbit, Abcam). We used ab56053 against CDA (1:500; Abcam) and β-actin (1:10000; Sigma).
Horseradish peroxidase-conjugated goat anti-mouse IgG and goat anti-rabbit IgG (Santa Cruz Biotechnology) were used at a dilution of 1:5000.

### Sister chromatid exchange assays

After treatment, cells were transferred to slides and cultured in the presence of 10 µM 5-bromodeoxyuridine (Sigma) at 37°C, under an atmosphere containing 5% CO₂. After 40 hours, Colchicine (Sigma) was added to a final concentration of 0.1 µg/ ml for 1 hour. Cells were incubated in hypotonic solution (1:6 (vol/vol) FCS-distilled water) and fixed with 3:1 (vol/vol) methanol-acetic acid. Cells were then stained by incubation with 10 µg/ ml Hoechst 33258 (Sigma) in distilled water for 20 minutes, rinsed with 2 x SSC (Euromedex), exposed to UV light at 365nm at a distance of 10 cm for 105 minutes, rinsed in distilled water, stained by incubation with 2 % Giemsa solution (VWR) for 16 minutes, rinsed in distilled water, dried and mounted. Chromosomes were observed with a Leica DMRB microscope at 100 x magnification. Metaphases were captured with a SONY DXC 930 P camera and SCEs or chromosomal aberrations were analyzed.

### Deoxyribonucleoside Triphosphates assay

The assay was performed as described in Sherman and Fyfe, Anal. Biochem 180, 222-6, 1989. Briefly, dNTPs were extracted from fresh cell pellets in trichloroacetic acid (TCA) 0.6M - MgC12 15mM. After centrifugation, TCA was extracted with trichlorofluomethane 78% - trioctylamine 22% (vol/vol), the aqueous phase containing dNTPs was recovered and used immediately for the assay or kept at -80°C.
For each dNTP assay, an oligonucleotide template primer had been designed (Sherman and Fyfe, Anal. Biochem 180, 222-6, 1989) and a DNA polymerization reaction (using 1 unit sequenase, 1 hour at 25°C) was achieved in the presence of the 3 other dNTPs in excess (i.e. 1µM - The dNTP assayed is by the way the limiting reagent) including radioactive labeled dATP (for dCTP, dTTP and dGTP assays) or radioactive labeled dTTP (for dATP assay). For each dNTP, a concentration standard range was performed from 0 to 4 pM.
At the end of the polymerization step, each sample was spotted on a DE81 membrane (Whatmann) that retains radioactive oligomers. After washes (3 x 10 minutes in Na₂HPO₄ 5%), and a final wash in water, membranes were dried and radioactivity was counted in scintillator. Sample concentration of each dNTP was then determined according to the standard range data.

### Results

### CDA expression is downregulated in BLM-deficient cells

We analyzed CDA expression at the mRNA and protein level in both BS cells and BLM-depleted HeLa cells. As shown in Figure 1a, CDA was not detectable in BS cells at both mRNA and protein level, whereas it expression was restored in BLM-complemented BS cells. siRNA-mediated downregulation of BLM in HeLa cells resulted in more that 2 times decrease in CDA expression (Fig. 1b). We constructed a non tumoral embryonic HEK cell line stably down-regulated for BLM expression using BLM specific shRNA and siRNAs (see material and methods section) and observed that mRNA expression of CDA was reduced near than 10 fold in these cells (Fig. 1c). These results demonstrate that CDA expression is strongly reduced in BLM-deficient cells.

### dCTP pool is significantly increased in BLM-deficient cells

CDA is an enzyme of the pyrimidine salvage pathway catalyzing the hydrolytic deamination of cytidine and deoxycytidine to the corresponding uridine and deoxyuridine (Nygaard, Adv. Exp. Med. Biol. 195, 415, 1986). Using an enzymatic assay, we measured deoxyribonucleoside triphosphates (dNTPs) pools in BS cells and found that they display a two to four fold increase in dCTP pool compared to BS cells complemented with a wild type BLM (Fig. 2). These results demonstrate that CDA defect lead to an accumulation of dCTP.

### Culturing BS cells in the presence of deoxyuridine (dU) or thymidine decreases significantly SCE level.

Since CDA irreversibly deaminates cytidine and deoxycytidine to their uridine counterparts, the CDA defect in BS cells is expected to be associated we a decrease in the dU and thymidine (T) pools (pools of dUTP and dTTP cannot be distinguished by the enzymatic assay that we used for dNTPs measures). Elevation of SCE levels being pathognomonic of BS cells, we analyzed the SCE frequency in BS cells cultured in the presence of 25 mM of dU or T. As shown in Figure 3, addition of dU or T in the culture medium led to a significant decrease in SCEs frequency in BS cells, whereas it did not affect SCEs frequency in BLM-complemented BS cells. These results establish a link between dNTP pool imbalance and the increase in SCE frequency in BS cells, and suggest that the CDA defect could participate in SCE formation in BS cells.

### siRNA-mediated depletion of CDA is associated with a significant increase in SCE frequency.

To further analyze the possible relationship between CDA defect and SCE formation, we transiently down-regulated CDA expression in BLM-complemented BS cells with a pool of siRNAs directed against CDA. CDA down-regulation was very efficient after two rounds of siRNA transfection, at both mRNA and protein level (Fig. 4A, upper and middle panels, respectively). CDA down-regulation was associated with a significant increase in SCE frequency in almost all cells and led to a 2.6 fold increase in the number of cells displaying more than 0.4 SCE per chromosome (Fig. 4A, lower panel). The same experiments were performed in HeLa cells and showed a significant increase in the number of cells presenting 0.3 to 0.4 SCE per chromosome (Fig. 4B). Finally, we further down-regulated CDA expression in HeLa cells down-regulated for BLM expression and observed an increase in the SCE frequency in the major part of cells, with a 2.3 increase in the number of cells exhibiting more than 0.4 SCE per chromosome (Fig. 4C). These results demonstrate that CDA down-regulation is associated with a significant increase in SCE frequency in control cells, and participates in the formation of SCEs in BLM-deficient cells.

### Conclusions

Here we show for the first time that siRNA-mediated down-regulation of CDA leads to an increase in SCE frequency. An elevation of the levels of SCEs is pathognomonic of Bloom syndrome, known to predispose patients to all types of cancers affecting the general population. Moreover, increases in SCEs levels in mammals have been used extensively to identify DNA-damaging carcinogens (Norppa, Toxicol. Lett. 149, 309, 2004). Thus, the increase in SCE frequency induced by CDA downregulation reflects a genetic instability that can predispose to cancer.

Moreover, our results showing that culturing BS cells in the presence of deoxyuridine reduces significantly their genetic instability indicate that individuals with CDA deficiency can be treated with dU, T, or other uridine analogs according to the invention.

### Example 2: Low CDA levels in cancer patients compared with the general population

### Material and methods

### Patients

Samples of peripheral blood are collected from 200 healthy donors and from 200 patients with different types of cancers as controls.

### CDA enzymatic activity

CDA status is evaluated by measuring residual enzymatic activity in serum, as described in Ciccolini et al., J Clinical Oncology, 28, 160-165, 2010. Briefly, cytidine conversion to ammonium is monitored spectrophotometrically after overnight incubation at 37°C. CDA activity is expressed as U/mg proteins, and 1U corresponds to 4.10⁻³ µMol of ammonium released per minute and per milliliter of serum. Proteins are assayed by using the standard Bradford method. Each sample is assayed in triplicate.

### RNA extraction and cDNA synthesis

Total RNA is extracted from the peripheral blood using TRIZOL Protocol for RNA extraction from blood according to the manufacturer's instructions. A DNAse digestion step is performed. RNA quality is tested using Experion system (Biorad) and 28S/18S ratio is calculated. If the ratio >1.8, RNA is used for cDNA synthesis. The cDNA are synthesized using 250 ng of random hexamers (Invitrogen), 2 µg RNA and Superscript II reverse transcriptase (Invitrogen) according to manufacturer's instructions.

### Quantitative Real-Time PCR experiments

The experiments are performed according to the MIQE Guideline (Bustin et al., Clin Chem 55, 611-22, 2009). Amplification mixtures (10µ1) contained template cDNA (1/100 final dilution), iQ SYBR Green Supermix 1x (BioRad) and 300 nM forward and reverse primer (see sequences in Example 1). Amplification is performed with CFX96 detection system (BioRad) in adapted 96 wells-plate (BioRad) and cycling conditions comprised 3 minutes polymerase activation at 95°C, and 40 cycles at 95° for 10 seconds and 60°C for 30 seconds. For each sample and primer set, the reactions are performed in triplicate. All PCR efficiencies are above 95% and below 102%.
The relative quantities of CDA are normalized, using CFX manager software (BioRad), against 4 reference genes mRNAs (HPRT1, RPL32, HMBS and SDHA, see Example 1), chosen because of their low M-value (Vandesompele et al., Genome Biol 3, 2002 - Calculated by the software).

## Claims

1. A method for detecting a predisposition to cancer in a patient comprising the step of detecting the level of cytidine deaminase (CDA) in a sample obtained from said patient.

2. A method according to claim 1 or 2, wherein said biological sample is a serum sample.

3. A method according to claim 1 or 2, wherein said level of CDA is the level of CDA enzymatic activity.

4. A method according to claim 1 or 2, wherein said level of CDA is the level of the CDA protein.

5. A method according to claim 1 or 2, wherein said level of CDA is the level of mRNA encoding CDA.

6. A method according to claim 1 or 2, wherein said level of CDA is assayed indirectly by genotyping the *CDA* gene.

7. A kit for detecting a predisposition to cancer in a patient according to any one of claims 1 to 6, wherein said kit comprises means for detecting the level of CDA in a biological sample obtained from said patient.

8. Use of a kit for detecting a predisposition to cancer in a patient, wherein said kit comprises means for detecting the level of CDA in a biological sample obtained from said patient.

9. An agent which increases the level of deoxyuridine for use in a method for preventing cancer in a patient.

10. An agent which increases the level of deoxyuridine according to claim 9, wherein said agent is an agent capable of restoring the level of the CDA protein.

11. An agent which increases the level of deoxyuridine according to claim 9, wherein said agent is a uridine analog.

12. An agent which increases the level of deoxyuridine according to claim 11, wherein said agent is a uridine analog selected from the group consisting of uracil, uridine, uridine monophosphate, uridine biphosphate, uridine triphosphate, thymine, thymidine, thymidine monophosphate, thymidine biphosphate, thymidine triphosphate, β-D-uridine-5'-bis(SATE)phosphotriester, triacetyluridine and 5-(phenylthio)acyclouridine.

13. A pharmaceutical composition comprising a uridine analog and another active agent and a pharmaceutically acceptable excipient.

14. A pharmaceutical composition according to claim 13, wherein said other active agent is vitamin D.

15. A pharmaceutical composition according to claim 13 or 14 for use in a method for preventing cancer in a patient.
